(19) Europäisches Patentamt
European Patent Office
Office européen des brevets

(11) Publication number: **0 068 617**
**A1**

(12) # EUROPEAN PATENT APPLICATION

(21) Application number: **82302483.1**

(22) Date of filing: **17.05.82**

(51) Int. Cl.³: **C 07 D 498/04**, A 61 K 31/42
//
(C07D498/04, 263/00, 205/00), (A61K31/42, 31/43),(A61K31/42, 31/545)

(30) Priority: **12.06.81 GB 8118108**
**17.09.81 GB 8128092**

(43) Date of publication of application: **05.01.83**
**Bulletin 83/1**

(84) Designated Contracting States: **BE CH DE FR GB IT LI NL SE**

(71) Applicant: **BEECHAM GROUP PLC, Beecham House Great West Road, Brentford Middlesex (GB)**

(72) Inventor: **Stirling, Irene, 38 Harrison Close, Reigate Surrey (GB)**
Inventor: **Clarke, Brian Peter, "Coneybury", Drive Spur Forest Drive, Kingswood Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Derivatives of clavulanic acid, a process for their preparation and their use.**

(57) Compounds of formula (I):

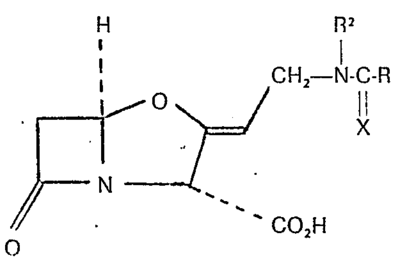

(I)

or a salt or ester thereof; wherein R represents a group $R^1$, $-OR^1$, $SR^1$ or $-NR_AR_B$, $R^1$, $R_A$ und $R_B$ independently represent hydrogen, a substituted or unsubstituted hydrocarbon radical, or $R_A$ and $R_B$ together form the residue of a heterocyclic ring, X represents oxygen or sulphur and $R^2$ represents an optionally substituted hydrocarbon or heterocyclic group; have antibacterial and β-lactamase inhibitory properties.

EP 0 068 617 A1

0068617

## β-LACTAM COMPOUNDS

This invention relates to β-lactam compounds and in particular to a class of clavulanic acid derivatives substituted by an acylamino group. These compounds have antibacterial and β-lactamase inhibitory qualities, and therefore are of use in the treatment of bacterial infection either alone or in a synergistic composition with other antibacterial agents, such as penicillins and cephalosporins.

The present invention provides a compound of the formula (I):

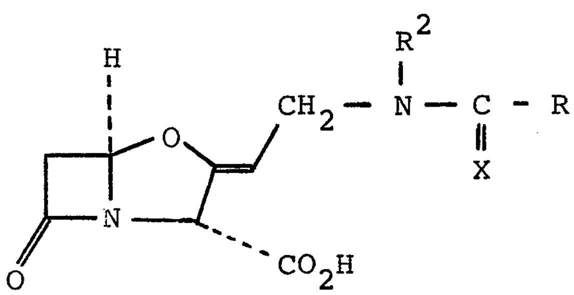

(I)

and salts and esters thereof; wherein R represents a group $R^1$, $-OR^1$, $SR^1$ or $NR_AR_B$, where $R^1$, $R_A$ and $R_B$ independently represent hydrogen, a substituted or unsubstituted hydrocarbon radical, or $R_A$ and $R_B$ together form the residue of a heterocyclic ring, X represents oxygen or sulphur and $R^2$ is an optionally substituted hydrocarbon or heterocyclic group.

The group $R^2$ may for example represent an optionally substituted $C_{1-6}$ alkyl group, a $C_{3-9}$ cycloalkyl group, or an optionally substituted heteroaryl methyl group.

Preferably X is an oxygen atom.

Suitably $R^1$ is hydrogen or an unsubstituted or substituted hydrocarbon radical of up to 20 carbon atoms. A preferred aspect of the invention is when X is oxygen, and R represents a group $R^1$.

In particular $R^1$ may be a $C_{1-6}$ alkyl, $C_{2-6}$ alkenyl, $C_{2-6}$ alkynyl, aryl $(C_{1-6})$ alkyl, aryl $(C_{2-6})$ alkenyl, heteroaryl $(C_{1-6})$ alkyl, $C_{3-8}$ cycloalkyl, aryl, heteroaryl, $C_{3-8}$cycloalkyl $(C_{1-6})$ alkyl, heterocyclyl or heterocyclyl $(C_{1-6})$ alkyl group, any of such groups being optionally substituted. Suitably the hetero atom or hetero atoms in the above named heteroaryl and/or heterocyclyl moieties are selected from 1 to 4 oxygen, nitrogen or sulphur atoms.

Suitable optional substituents for the group $R^1$ include $C_{1-6}$ alkyl, amino, $C_{1-6}$ alkanoylamino, mono, di- and tri- $(C_{1-6})$ alkylamino, hydroxy, $C_{1-6}$ alkoxy, mercapto, $C_{1-6}$ alkylthio, heteroarylthio, arylthio, oxo, sulphamoyl, carbamoyl, amidino, guanidino, nitro, chloro, bromo, fluoro, carboxy and salts and esters thereof, $C_{1-6}$ alkanoyloxy, arylcarbonyl and heteroarylcarbonyl.

Most suitably $R^1$ is a $C_{1-6}$ alkyl group, for example a methyl, ethyl, propyl or butyl group. Suitably also $R^1$ is a $C_{2-6}$ alkenyl group such as an allyl group. $R^1$ may also be an aryl ($C_{1-6}$) alkyl group preferably where the aryl group is phenyl, for example phenethyl, benzyl and substituted benzyl where preferably the substituents are hydroxy, acetoxy, amino and carboxy wherein such substituents may be on the ring or the methylene carbon. In an alternative $R^1$ may be an aryl or heteroaryl group such as phenyl or substituted phenyl, for example hydroxyphenyl, chlorophenyl, $C_{1-3}$ alkylphenyl or $C_{1-3}$ alkoxyphenyl. Suitably also $R^1$ may be a $C_{3-8}$ cycloalkyl ring such as cyclopropyl, cyclobutyl, cyclopentyl or cyclohexyl. Suitably the heteroaryl moieties referred to hereinabove contain from 5 to 6 ring atoms attached via a carbon atom to the -C(=X)- moiety, such as furyl, thienyl, pyridyl and pyrrolyl. Suitably the heterocyclyl moieties referred to hereinabove contain from 4 to 8 ring atoms attached via a carbon atom to the -C(=X)- moiety, such as azetidine, tetrahydropyran and tetrahydrofuran. In a further aspect it is to be realised that any heteroaryl or aryl moiety may be fused so as to form a bicyclic fused ring system, for example indolyl, naphthyl, benzofuryl and quinolyl.

Suitably the group $R^2$ contains from 1 to 14 carbon atoms, in particular from 1 to 8 carbon atoms. The group $R^2$ may be substituted with, for example, halogen, hydroxy, cyano, carboxylic acid, salt or ester, $OR^C$, $SR^C$, $COR^C$, $OCOR^C$ or $OCO_2R^C$, where $R^C$ is a hydrocarbon group of from 1 to 8 carbon atoms. Suitably $R^2$ is a $C_{1-6}$ alkyl group optionally substituted by hydroxy, carboxy or salts or esters thereof, cyano, a heterocyclyl ring bonded _via_ a nitrogen atom, or a group of the sub-formula i):

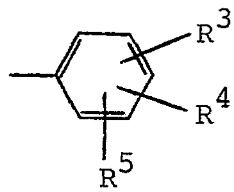

i)

wherein $R^3$ is a hydrogen, fluorine, chlorine or bromine atom or a $C_{1-3}$ alkyl, $C_{1-3}$ alkoxy, $C_{1-3}$ alkanoyloxy, hydroxy, $C_{1-4}$ alkoxycarbonyl group, or a group $-N(R^6)COR^7$, $-N(R^6)SO_2R^7$ or $-CONR^6R^7$ wherein $R^6$ is a hydrogen atom, $C_{1-3}$ alkyl, phenyl or benzyl and $R^7$ is $C_{1-3}$ alkyl, phenyl or benzyl; $R^4$ is a hydrogen, fluorine or chlorine atom or is $C_{1-3}$ alkyl, $C_{1-3}$ alkoxy or $C_{1-3}$ alkanoyloxy; and $R^5$ is a hydrogen, fluorine or chlorine atom or a $C_{1-3}$ alkoxy or alkyl group. Suitably also $R^2$ is a $C_{3-9}$ cycloalkyl group, for example cyclopropyl, cyclobutyl, cyclopentyl and cyclohexyl, of these cyclopropyl is favoured.

Suitably also $R^2$ is an optionally substituted heteroarylmethyl group wherein the heteroaryl moiety is an aromatic monocyclic or bicyclic heterocyclic group bonded via a carbon atom. Suitable aromatic monocyclic heterocyclic groups are those containing 5 or 6 ring atoms. Suitable aromatic bicyclic heterocyclic groups are those having a benzene ring fused to an aromatic heterocyclic group containing 5 or 6 ring atoms. Thus suitable groups $R^2$ include the furylmethyl, thienylmethyl, pyrrolylmethyl N-methylpyrrolylmethyl, indolylmethyl, oxazolylmethyl, thiazolylmethyl and quinolylmethyl groups. Suitable substituents include those hereinbefore stated to be suitable for $R^1$.

More suitably $R^2$ is a $C_{1-6}$ alkyl group optionally substituted by hydroxy, carboxy or salts or esters thereof or cyano, for example $R^2$ may be $C_{1-6}$ alkyl either branched or straight-chain such as methyl, ethyl, n-propyl, isopropyl, n-butyl sec-butyl, isobutyl, pentyl or hexyl, of these ethyl, n-propyl, and isobutyl are favoured with isobutyl being preferred; alternatively $R^2$ may be $C_{1-6}$ alkyl substituted by carboxy, for example carboxyethyl, carboxypropyl and carboxybutyl, or $R^2$ may be $C_{1-6}$ alkyl substituted by cyano such as cyanoethyl, or $R^2$ may be $C_{1-6}$ alkyl substituted by hydroxy such as hydroxyethyl.

Suitably also $R^2$ is a $C_{1-6}$ alkyl group, preferably a methyl group substituted by a group of the sub-formula i) as hereinbefore defined. Thus favoured groups $R^2$ include benzyl and benzyl substituted by one, two or three groups or atoms selected from fluorine, chlorine, bromo, $C_{1-3}$ alkyl, $C_{1-3}$ alkoxy, hydroxy, $C_{1-6}$ alkanoylamino such as acetamido, and $C_{1-4}$ alkoxycarbonyl such as methoxycarbonyl. Preferably $R^2$ is benzyl, hydroxybenzyl, acetamidobenzyl and methoxybenzyl.

Suitably also $R^2$ is a $C_{1-6}$ alkyl group substituted by a heterocyclyl ring bonded <u>via</u> a nitrogen atom to said alkyl group. Suitably the heterocyclyl ring contains 3 to 8 ring atoms, one of which (in addition to the above mentioned nitrogen atom) may be selected from nitrogen, oxygen or sulphur. Said heterocyclyl ring may be substituted by one or two groups selected from $C_{1-6}$ alkyl, $C_{1-6}$ alkoxy, hydroxy, oxo or phenyl. Thus suitable values for $R^2$ include morpholino $(C_{1-6})$ alkyl such as morpholinopropyl and morpholinoethyl, and pyrrolidino $(C_{1-6})$ alkyl such as pyrrolidinopropyl.

Suitably the group $NR^AR^B$ contains up to 16 carbon atoms. Suitable values of $R_A$ are as described hereinabove for the group $R^1$. Suitable values of $R_B$ are as described hereinabove for the group $R^1$.

A group of compounds of interest is that wherein X is oxygen, and R is hydrogen, $C_{1-6}$ alkyl, phenyl, $C_{1-6}$ alkylaminocarbonyl, aminocarbonyl or di- $(C_{1-6})$ alkylaminocarbonyl.

Another group of compounds of interest is that wherein X is oxygen, and R is $C_{1-6}$ alkoxy example methoxy or ethoxy, or is phenoxy.

A further group of interest is that wherein X is oxygen or sulphur, and R is hydrogen, $C_{1-6}$ alkyl, $C_{1-6}$ alkoxy, $C_{1-6}$ alkylamino or phenyl optionally substituted with up to three substituents selected from $C_{1-6}$ alkyl, nitro, halogen or hydroxy.

Thus a favoured sub-group of compounds of this invention is that of the formula (II):

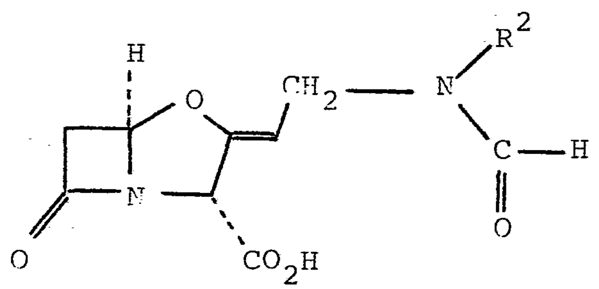

(II)

and salts and esters thereof wherein $R^2$ is as hereinbefore described.

A preferred class of compounds of this invention is that of the formula (III):

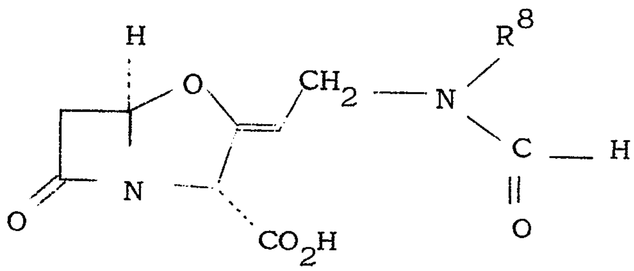

(III)

and salts and esters thereof wherein $R^8$ is $C_{3-6}$ cycloalkyl, such as cyclopropyl; $C_{1-6}$ alkyl, such as methyl, ethyl, n-propyl, isopropyl, n-butyl, sec-butyl and isobutyl; or carboxy ($C_{1-6}$) alkyl, such as carboxy-n-propyl. Of these ethyl, n-propyl and isobutyl are favoured.

Thus particular compounds of this invention include: 9-(N-cyclopropyl)formamidodeoxyclavulanic acid, 9-[N-(3-carboxypropyl)] formamidodeoxyclavulanic acid, 9-(N-ethyl) formamidodeoxyclavulanic acid, 9-(N-isobutyl)formamidodeoxyclavulanic acid, 9-N-(isobutyl)-N-(ethoxycarbonyl)amino-deoxyclavulanic acid, 9-N-(isobutyl)-N-(benzoyl)amino-deoxyclavulanic acid, 9-[N-isobutyl-N'-methylureido] deoxyclavulanic acid, 9-[N-isobutylureido] deoxyclavulanic acid, 9-[N-isobutyl-N'-methylthioureido] deoxyclavulanic acid, and 9-N-(isobutyl)-N-(acetyl)aminodeoxyclavulanic acid, and pharmaceutically acceptable salts thereof.

The major utility of the compounds of the formulae (I)-(III) and salts and esters thereof is as pharmaceuticals and accordingly the salts and esters of the compounds of the formulae (I) - (III) are preferably pharmaceutically

BAD ORIGINAL

acceptable. The compounds of this invention both pharmaceutically acceptable and non-pharmaceutically acceptable may be used as intermediates and also as antibacterial agents or β-lactamase inhibitors in non-pharmaceutical usage such as a disinfectant or paint additive.

Suitable pharmaceutically acceptable salts of the compounds of the formula (I)-(III) include metal salts such as aluminium, alkali metal salts such as sodium and potassium, and alkaline earth metal salts such as calcium or magnesium; and ammonium and substituted ammonium salts, for example those with lower alkylamines such as triethylamine, cycloalkylamines such as bicyclohexylamine, or with dibenzylamine, N,N-dibenzylethylenediamine, 1-ephenamine, N-ethylpiperidine and N-benzyl-β-phenethylamine.

Suitable esters of the compounds of the formulae (I) - (III) include those cleavable by biological methods such as enzymatic hydrolysis, in-vivo hydrolysis, and those cleavable by chemical methods such as hydrogenolysis, hydrolysis, electrolysis or photolysis.

Suitably the carboxylic acid is esterified by a group of the sub-formula (a), (b), (c), (d), (e), or (f):

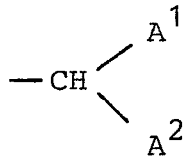 (a)

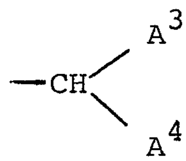 (b)

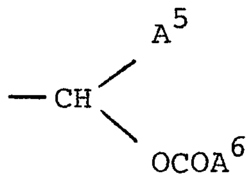 (c)

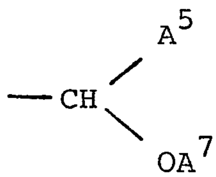 (d)

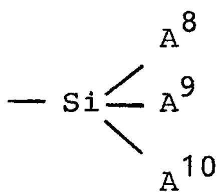 (e)

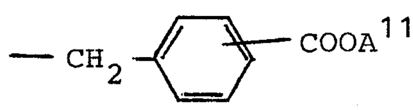 (f)

wherein $A^1$ is a hydrogen atom, $C_{1-6}$ alkanoyl or an $C_{1-5}$ alkyl group optionally substituted by $C_{1-7}$ alkoxy or $C_{1-7}$ carboxylic acyloxy, or an alkenyl or alkynyl group of up to 5 carbon atoms; A2 is a hydrogen atom or a methyl group; $A^3$ is a phenyl group or a phenyl group substituted by a fluorine, chlorine or bromine atom or a nitro, $C_{1-3}$ alkyl or $C_{1-3}$ alkoxy group; $A^4$ is a hydrogen atom or a phenyl group or phenyl group substituted by a fluorine,

chlorine or bromine atom or a nitro, $C_{1-3}$ alkyl or $C_{1-3}$ alkoxy group; $A^5$ is a hydrogen atom or a methyl group; $A^6$ is a $C_{1-4}$ alkyl, phenyl or $C_{1-4}$ alkoxy group or $A^5$ is joined to $A^6$ to form a phthalidyl, dimethylphthalidyl or dimethoxy-phthalidyl group; $A^7$ is a $C_{1-4}$ alkyl, phenyl chlorophenyl or nitrophenyl group; $A^8$ is a $C_{1-4}$ alkyl or phenyl group; $A^9$ is a $C_{1-4}$ alkyl or phenyl group; $A^{10}$ is $C_{1-4}$ alkyl; and $A^{11}$ is $C_{1-4}$ alkyl: or $CHA^1A^2$ is a phenacyl or bromo phenacyl group.

Favourably $A^1$ is a hydrogen atom or a methyl, ethyl, vinyl or ethenyl group. Favourably $A^2$ is a hydrogen atom. Favourably $A^3$ is a phenyl, p-bromophenyl, p-methoxyphenyl or p-nitrophenyl group. Favourably $A^4$ is a hydrogen atom. Favourably $A^6$ is a methyl, t-butyl or ethoxy group or is joined to $A^5$. Favourably $A^7$ is a methyl group.

Preferred groups of the sub-formula (a) include the methyl, ethyl and acetonyl groups.

Preferred groups of the sub-formula (b) include the benzyl and p-nitrobenzyl groups.

Preferred groups of the sub-formula (c) include the acetoxymethyl, pivaloyloxymethyl, α-ethoxycarbonyl-oxymethyl and phthalidyl groups.

A preferred group of the sub-formula (d) is the methoxymethyl group.

Preferred groups of the sub-formula (e) include the trimethylsilyl, tert-butyldimethylsilyl and tert-butyldiphenylsilyl groups.

A preferred group of the sub-formula (f) is p-methoxycarbonylbenzyl.

Particularly preferred esterifying groups are the p-nitrobenzyl and phthalidyl groups.

Pharmaceutically acceptable in vivo hydrolysable esters are those esters which hydrolyse in the human body to produce the parent acid or its salt. Such esters may be identified by administration to a test animal such as a rat or mouse by intravenous administration and thereafter examining the test animal's body fluids for the presence of the compound of the formulae (I)-(III) or salt.

Suitable esters of this type include those of sub-formula (c) as hereinbefore defined.

Further suitable esters include di($C_{1-6}$) alkylamino $C_{1-6}$ alkyl esters such as dimethylaminomethyl, dimethylaminoethyl, diethylaminomethyl and diethylaminoethyl.

Esters of the compounds of the formulae (I)-(III) such as those of the sub-formula (a)-(f) if desired, may be presented in the form of their acid addition salts if an amino group is present in either of groups $R^1$ or $R^2$. The acid used to form the salt will most suitably be pharmaceutically acceptable, but non-pharmaceutically acceptable acid addition salts are also envisaged, for example as intermediates in the preparation of the pharmaceutically acceptable salts by ion exchange.

Suitable pharmaceutically acceptable acid addition salts include those of inorganic and organic acids, such as hydrochloric, phosphoric, sulphuric, methanesulphonic, toluenesulphonic, citric, malic, acetic, lactic, tartaric, propionic and succinic acid.

Most suitably the acid addition salt is provided as a solid and preferably as a crystalline solid.

Compounds of this invention when in crystalline form may be solvated, for example hydrated.

The present invention provides a pharmaceutical composition which comprises a compound of this invention and a pharmaceutically acceptable carrier.

The compositions of the invention include those in a form adapted for oral, topical or parenteral use and may be used for the treatment of the infection in animals for example mammals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating antibiotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water: sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam antibiotics for inclusion in such synergistic compositions include not only those known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, piperacillin, and other known penicillins including pro-drugs therefor such as their *in vivo* hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamido side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or cephalosporin present in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particules of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios, such as from 10:1 to 1:10 for example about 3:1, 2:1, 1:1, 1:2, 1:3, 1:4, 1:5 or 1:6, (wt/wt, based on pure free antibiotic equivalent). Orally administrable compositions containing a compound of the

invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

Compositions of this invention may be used for the treatment of infections of inter alia the respiratory tract, the urinary tract and soft tissues in humans and mastitis in cattle.

Normally between 50 and 3000 mg of the compounds of the invention will be administrered each day of treatment but more usually between 100 and 1000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillinhydrochloride, bacampicillin hydrochloride, or talampicillin hydrochloride. Most suitably this form of the composition will contain a compound of this invention when in crystalline form.

Most suitably the preceding composition will contain from 200 to 700 mg of the penicillin component. Most suitably the preceding composition will comprise from 50 to 250 mg of a compound of this invention preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefo and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of this invention preferably in crystalline form. Such compositions containing di-salts of carbeniclln and ticarcillin will be adapted for parenteral administration.

The present invention also provides a method of treating bacterial infections in humans or domestic mammals which comprises the administration of a composition of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an anti-bacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of the formula (I) or a salt or ester thereof, which process comprises the reaction of a compound of the formula (IV):

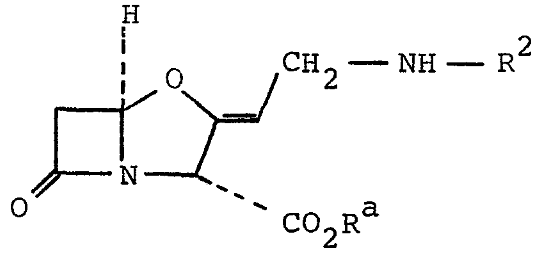

(IV)

wherein $R^2$ is as hereinbefore defined and $R^a$ is a hydrogen atom or a carboxy-blocking group; with an N-acylating derivative of an acid of formula R.COH,
X

wherein R and X are as hereinbefore defined and optionally thereafter

i) removing any carboxy-blocking group $R^a$,
ii) converting the product into a salt or ester,

Suitable N-acylating derivatives include any N-acylating compound suitable for the performance of analogous reactions with 6-aminopenicillanic acid or 7-aminocephalosporanic acid or salts or esters thereof, for example an acid halide, an anhydride or mixed anhydride or other reactive derivative, such as that produced by the reaction of an acid with an enzyme or a condensation-promoting agent such as dicyclohexylcarbodiimide.

Suitably the N-acylating derivative is a compound of the formula (V):

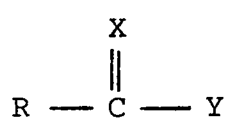

(V)

wherein $R^1$ and X are as hereinbefore defined, and Y is a readily displaceable group. Suitably Y is halo such as chloro or bromo, or a sulphonyloxy moiety, for example $C_{1-6}$ alkyl or arylsulphonyloxy, such as a mesylate or a tosylate, or Y is a carboxylate moiety, for example a $C_{1-6}$ alkanoyloxy group optionally substituted by 1 to 3 halogen atoms, in particular acetoxy, dichloroacetoxy and trichloroacetoxy, or an arylcarbonyloxy moiety for example benzoyloxy. Alternatively Y is a hydroxy group or hydrocarbyloxy group, for example $C_{1-6}$ alkoxy or aryloxy such as phenoxy.

Preferably the reaction is performed in the presence of a base, for example an inorganic base such as an alkali or alkaline earth metal salt, in particular lithium, sodium or potassium carbonates; or an organic base such as an amine, for example a tertiary amine and in particular triethylamine.

Suitably the reaction is performed in an aprotic organic solvent such as dimethylformamide, acetonitrile, ethyl acetate, dimethyl sulphoxide, diethyl ether, tetrahydrofuran, dichloromethane, chloroform or mixtures thereof. Water may be added to aid solubility, a particularly preferred solvent mixture is aqueous dimethylformamide. The reaction is suitably performed at a non-extreme temperature, for example between $-20^oC$ and $+40^oC$ and preferably at ambient.

Alternatively when it is desired to form a compound wherein R represents a group $-NR_AR_B$, suitable acylating agents include isocyanates and thioisocyanates, preferably such reaction is performed in an aprotic solvent at a non-extreme temperature.

Suitable carboxyl-blocking derivatives for the group $-CO_2R^a$ in formulae (IV) include salts, esters, and anhydride derivatives of the carboxylic acid. The derivative is one which may readily be cleaved at a later stage of the reaction. The salts need not be pharmaceutically acceptable. Suitable salts include inorganic salts, for example metal salts such as silver or mercuric salt, or alkali metal salts such as the lithium or sodium salt, and tertiary amine salts.

Suitable ester-forming carboxyl-blocking groups are those which may be removed under conventional conditions, and include those of sub-formulae (a)-(f) as hereinbefore defined. Such groups for $R^a$ include benzyl, p-methoxybenzyl, 2,4,6-trimethylbenzyl, 3,5-dit-butyl-4-hydroxybenzyl, benzoylmethyl, p-nitrobenzyl, 4-pyridyl-methyl, 2,2,2-trichloroethyl, 2,2,2-tribromoethyl, acetonyl, 2-methylallyl, t-butyl, t-amyl, diphenylmethyl, triphenylmethyl, methyl, ethyl, allyl, adamantyl, 2-benzyloxyphenyl, 4-methylthiophenyl, tetrahydrofur-2-yl, tetrahydropyran-2-yl, methoxymethyl, a silyl, stannyl or phosphorus-containing group, or an <u>in vivo</u> hydrolysable ester.

The carboxyl group may be regenerated from any of the above esters by usual methods appropriate to the particular $R^a$ group, for example, acid - and base - catalysed hydrolysis, or by enzymically - catalysed hydrolysis, photolysis or by hydrogenation. The hydrolysis must of course be carried out under conditions to which the groups on the rest of the molecule are stable.

When it is desired to produce a compound of formula (I) in the form of a free acid or salt by the process of this invention, a compound of formula (IV) is generally employed wherein $R^a$ is a carboxyl-blocking group. For the preparation of a compound of formula (I) in the form of a pharmaceutically acceptable ester, it is convenient to employ a compound of formula (IV) wherein $R^a$ represents the desired ester group.

The compounds of the formula (IV) may be prepared by the methods of European Patent Application Publication Numbers: 5014, 7717, 9302, 8884, 21836, 25271, 27323, 27324 and Belgian Patent Number 866276.

The following Examples illustrate the invention.

Example 1

Lithium 9(N-cyclopropyl)formamidodeoxyclavulanate

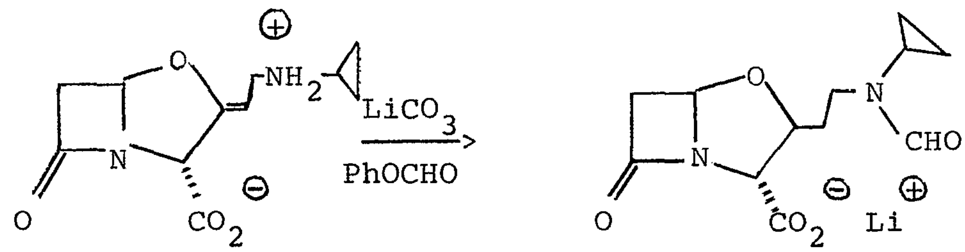


9-N-Cyclopropylaminodeoxyclavulanic acid (1.11g; 5.24 mmol) in dimethylformamide (20 $cm^3$) and water (12 $cm^3$) was treated with $^1/2$ equivalent lithium carbonate (0.193g) followed by 1.2 equivalents of phenyl formate and the mixture stirred at room temperature for five hours. The reaction mixture was allowed to stand at $-10^o$ for 15 hours after which the solvent was removed by evaporation under high vacuum. The residue was chromatographed on silica eluting with ethylacetate - isopropylalcohol - water (5:4:2) and fractions containing the title compound collected Rf ($SiO_2$/solvent system as above) = 0.61. Combined fractions were evaporated and triturated with acetone to afford a white solid, yield = 0.63g (44%). υ(nujol) 1785, 1660, 1620, 1305, 1190, 1120, 1040, 900, 740 $cm^{-1}$; υ(KBr) 1788, 1680 (very broad), 1400, 1360, 1308, 1190, 1118, 1040, 1027, 900, 740 $cm^{-1}$; δ($D_2O$)

0.58-0.88 (4H, m, 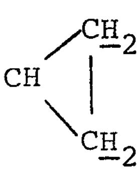 ), 2.53-2.83

(1H, m, 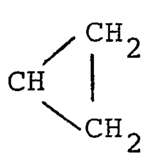 ), 3.01 (1H, d, J 17 Hz, 6βCH),

3.51 (1H, dd, J 17 and 3 Hz, 6αCH), 3.83-4.03 (2H, m, $9CH_2$), 8CH obscured by HOD, 4.87 (1H, broad s, 3CH), 5.65 (1H, d, J 3 Hz, 5αCH), 7.99 and 8.07 (1H, 2 x s, NCHO).

Example 2

Dilituium 9[N-(3-carboxypropyl)] formamidodeoxyclavulanate

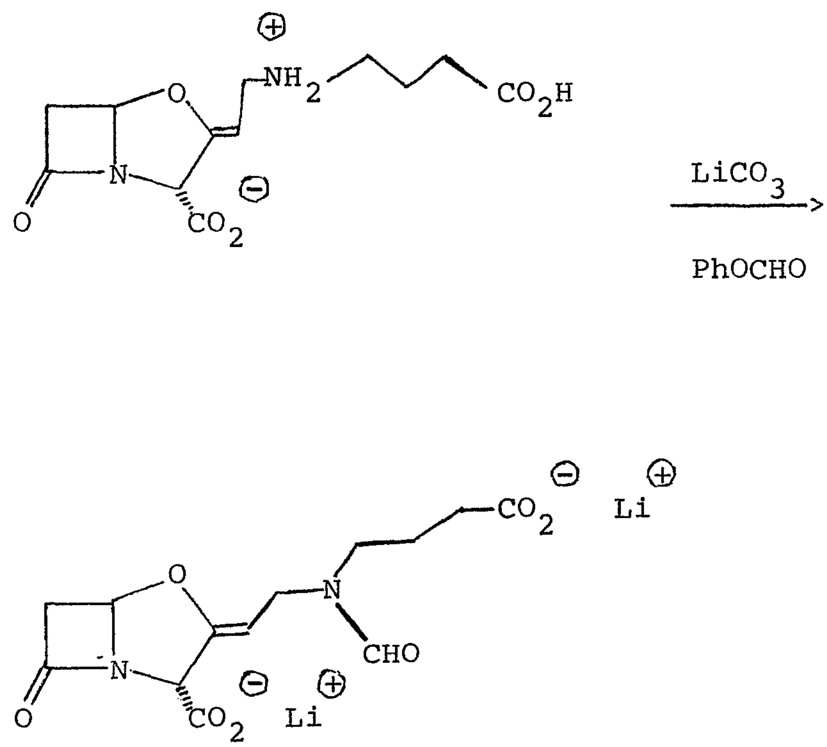


9-N-(3-Carboxypropyl)aminodeoxyclavulanic acid (0.528g; 1.87 mmol) in dimethylformamide (10 $cm^3$) and water (7 $cm^3$) was treated with one equivalent (138 mg) of lithium carbonate with stirring, followed by phenyl formate (12.2 equivalents). The mixture was stirred for 26 hours, when the solvent was evaporated under high vacuum and the residue chromatographed on silica eluting with ethylacetate-ethanol-water (5:3:2). Fractions were collected containing the title compound Rf ($SiO_2$/ethylacetate-ethanol-water; 5:3:2) = 0.6. Combined fractions were evaporated and on addition of ethanol a white solid formed. This solid was filtered off cold, washed with cold ethanol and dried to afford 230 mg (38%) of a white

solid. υ(nujol) 1785, 1650 (very broad). υ(KBr) 1786, 1645 (vbr), 1400, 1305, 1187, 1115, 1040, 1015, 895 $cm^{-1}$. δ($D_2O$) 1.55-1.95 (2H, broad m, $NCH_2C\underline{H}_2CH_2CO_2$), 2.13 (2H, broad t, _J_ 6.5 Hz, $NCH_2CH_2C\underline{H}_2CO_2$), 3.00 (1H, d, _J_ 17 Hz, 6βC$\underline{H}$), 3.08-3.35 (2H, broad m, $NC\underline{H}_2CH_2CH_2$), 3.50 (1H, dd, _J_ 17 and 3 Hz, 6αC$\underline{H}$), 3.92 (2H, broad d, _J_ 7 Hz, $9C\underline{H}_2$), 4.72 (1H, broad t, _J_ 7 Hz, 8C$\underline{H}$), 4.88 (1H, s, 3C$\underline{H}$, 5.68 (1H, broad d, _J_ 3 Hz, 5αC$\underline{H}$), 7.89 and 7.96 (1H, 2 x s, NC$\underline{H}$O).

Example 3

Lithium 9-(N-ethyl)formamidodeoxyclavulanate

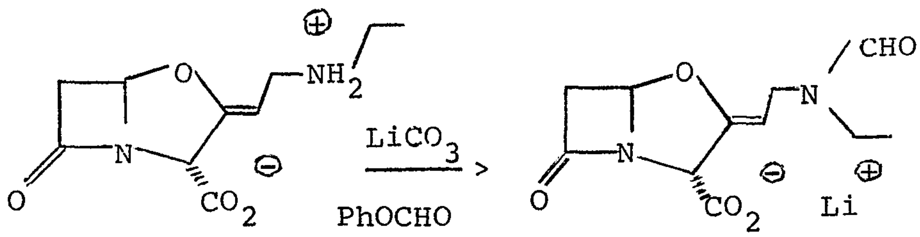


9-N-Ethylaminodeoxyclavulanic acid (1.29g; 5.70 mmol) in dimethylformamide (20 $cm^3$) and water (10 $cm^3$) was treated with $^1/2$ equivalent of lithium carbonate followed by 1.2 equivalents of phenyl formate and stirred for 24 hours. The solvent was removed under high vacuum and the residue chromatographed on silica eluting with ethyl acetate - ethanol - water (5:3:3) and fractions containing the title compound were collected Rf ($SiO_2$/ethylacetate - isopropylalcohol - water; 5:4:2) = 0.5 (detection by aqueous potassium permanganate spray). Combined fractions were evaporated to an oil, triturated with acetone and filtered off. Drying afforded 720 mg (49%) of a white solid. $\nu$(nujol) 1785, 1655, 1615, 1300, 1190, 1115, 1080, 1035, 1015, 895 $cm^{-1}$; $\delta$($D_2O$) 1.03 and 1.11 (3H, 2 x t, J 7Hz, $NCH_2CH_3$), 3.03 (1H, d, J 17Hz, 6βCH), 3.18-3.40 (2H, m, $NCH_2CH_2$), 3.52 (1H, dd, J 17 and 3Hz, 6αCH), 3.95 (2H, d, J 7Hz, $9CH_2$), 4.74 (1H, broad t, J 7Hz, 8CH), 4.88 (1H, broad s, 3CH), 5.69 (1H, d, J 3 Hz, 5αCH), 7.95 (1H, s, NCHO).

Example 4

Lithium 9-(N-isobutyl)formamidodeoxyclavulanate

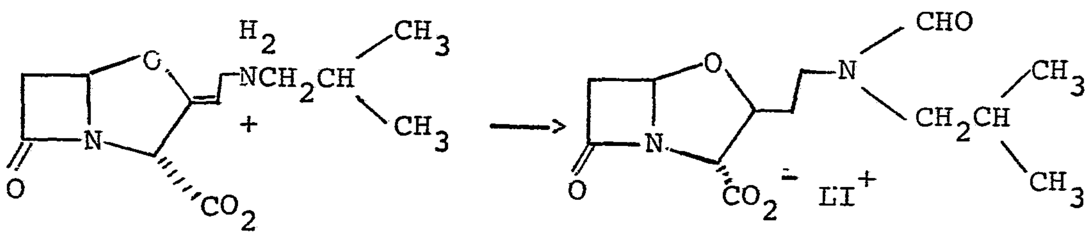

A suspension of 9-N-isobutylaminodeoxyclavulanic acid (254 mg; 1 mmol) in dimethylformamide (10 ml) was treated with a solution of lithium carbonate (37 mg; 0.5 mmol) followed by phenyl formate (134 μl; 1.2 mmol); the resulting solution was stirred at room temperature for 8 hours. The dimethylformamide was removed *in vacuo* and the residual solid was fractionated on silica using ethyl acetate : propan-2-ol : water (5:4:2) as eluent. Fractions containing the required product, Rf ($SiO_2$/ ethyl acetate : propan-2-ol : water; 5:4:2) = 0.61, were combined and evaporated *in vacuo*; the title compound was obtained as a colourless crystalline solid from acetone. Yield 73%.

$\upsilon_{max}$(Nujol) 1785, 1690 (sh), 1600-1660 $cm^{-1}$; $\upsilon_{max}$ (KBr) 1785, 1685 (sh), 1675, 1658, 1615 $cm^{-1}$. $\delta$ ($D_2O$) 0.79 (6H, d, *J* 7Hz, $CH(C\underline{H}_3)_2$), 2.0 (1H, m, $C\underline{H}(CH_3)_2$), 3.0 (1H, d, *J* 17 Hz, 6β-C$\underline{H}$), 3.03 (2H, d, *J* 7Hz, $C\underline{H}_2$-$CH(CH_3)_2$), 3.53 (1H, dd, *J* 17 and 3 Hz, 6α-C$\underline{H}$), 3.96 (2H, d, *J* 7 Hz, 9-$C\underline{H}_2$), 4.68 (1H, m, 8-C$\underline{H}$), 4.88 (1H, d, *J* 1.5 Hz, 3-C$\underline{H}$), 5.69 (1H, d, *J* 3Hz, 5-C$\underline{H}$), 7.93, 8.08 (1H, both singlets, N.C$\underline{H}$O).

Example 5

Lithium 9-N-(isobutyl)-N-ethoxycarbonyl)amino deoxyclavulanate

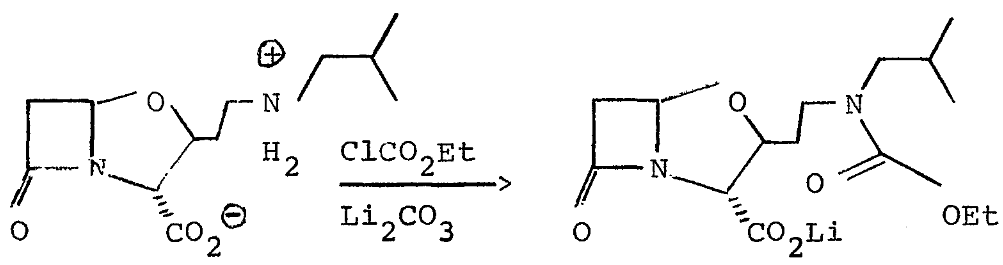


To a solution of 9-N-isobutylaminodeoxyclavulanic acid (0.8g; 3 mmol) in dimethylformamide (15 ml) and water (12 ml) at $0^oC$ was added lithium carbonate (0.12g) and ethyl chloroformate (0.31 ml; 3.2 mmol). The reaction mixture was stirred at $0^oC$ for 2½ hours then poured on to water (70 ml). The aqueous layer was washed with ethyl acetate (2 x 70 ml) and the solvent removed under reduced pressure to give an off-white solid. This solid was chromatographed on silica, eluting with ethyl acetate - ethanol - water (4:1:½), and the fractions containing the required product, Rf. 0.34, (ethyl acetate - ethanol - water; 4:1:½) were combined and evaporated in vacuo; the title compound was obtained as a white solid after precipitation from acetone with ether, (0.73g, 72%).

$\nu$max (KBr). 1790, 1680 (broad), 1600 (broad) $cm^{-1}$.

$\delta_H(D_2O)$, 5.66 (1H, d, J=3Hz, $H-C_5$), 4.85 (1H, S, $H-C_3$), 4.75 (1H, broad t, J=7Hz, $H-C_8$), 4.05 (2H, Q, J=7Hz, $OCH_2CH_3$), 3.89 (2H, d, J=7 Hz, $H_2-C_9$), 3.55 (1H, dd,

J=3Hz and 17 Hz, αH-$C_6$), 3.02 (2H, d, J=7Hz, $CH_2CH(CH_3)_2$), 2.95 (1H, d, J=17Hz, βH-$C_6$), 1.85 (1H, broad m, $CH(CH_3)_2$), 1.22 (3H, t, J=7Hz, $OCH_2CH_3$) 0.83 (6H, d, J=7Hz, $CH(CH_3)_2$).

Example 6

Benzyl 9-N-(isobutyl)-N-(benzoyl)aminodeoxyclavulanate

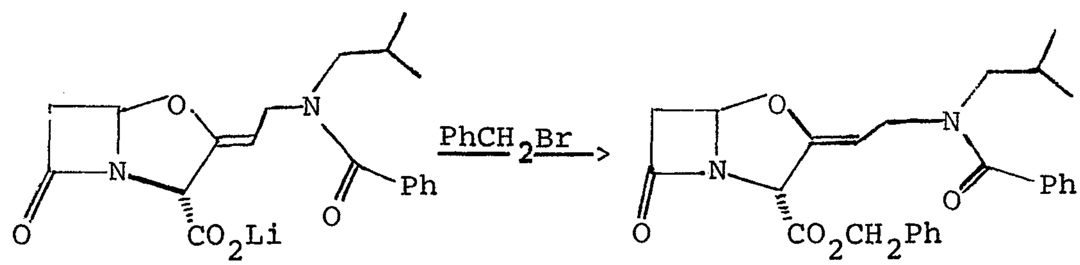


Lithium 9-N-(isobutyl)-N-(benzoylaminodeoxy-clavulanate (0.12g; 3 mmol) and benzyl bromide (0.04 ml., 3.4 mmol) were dissolved in dimethylformamide (5 ml) and stirred at room temperature for 7 hours. The reaction mixture was then poured on to ethyl acetate (30 ml) and the resulting organic layer washed with water (2 x 30 ml) and saturated sodium chloride solution (30 ml) before being dried over anhydrous magnesium sulphate. Removal of the solvent under reduced pressure yielded a colourless oil which was chromatographed on silica, eluting with ethyl acetate - cyclohexane (1:1). The fractions containing the component of Rf (ethyl acetate : cyclohexane; 1:1) = 0.46 were combined and the solvent removed under reduced pressure to yield the title compound as a colourless oil (0.05g, 33%).

$\nu$ max ($CHCl_3$) 1800, 1750, 1690, 1620 $cm^{-1}$.

$\delta_H$ 55°C ($CDCl_3$) 7.30 (10 H, S, $-CH_2$-Ph and COPh), 5.56

(1H, d, J=3Hz, H-$C_5$), 5.16 (2H, S, -$CH_2$Ph), 5.02 (1H, S, H-$C_3$), 4.66 (1H, broad t, J=7Hz, H-$C_8$), 4.02 (2H, d, J = 7Hz, $H_2$-$C_9$), 3.44 (1H, dd, J = 3Hz and 17 Hz, αH-$C_6$), 3.10 (2H, d, J = 7Hz, $CH_2CH(CH_3)_2$), 2.88 (1H, d, J = 17Hz, βH-$C_6$), 1.95 (1H, broad m, $CH(CH_3)_2$), 0.77 (6H, d, J = 7Hz, $CH(CH_3)_2$).

Example 7

Lithium 9(N-isobutyl-N'-methylureido) deoxyclavulanate

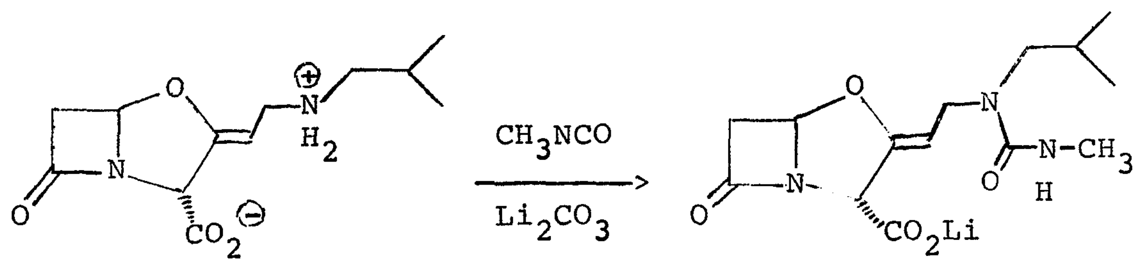


9-N-isobutylaminodeoxyclavulanic acid (0.2g; 0.8 mmol), was stirred at room temperature in dimethylformamide (10 ml) containing water (5 ml) and lithium carbonate (0.03 g) for 1/4 hr. The reaction mixture was then filtered, and the filtrate evaporated in vacuo to yield lithium 9-N-isobutylaminodeoxyclavulanate (0.18 g) as a pale yellow solid, crystallised from acetone as colourless crystals. (0.16 g).

To lithium 9-N-isobutylaminodeoxyclavulanate (0.16g; 0.6 mmol) in dry redistilled dimethylformamide (10 ml) was added methylisocyanate (0.04g, 0.7 mmol). The reaction mixture was stirred at room temperature for 3 hours, then poured on to water (20 ml). The aqueous layer was washed with ethyl acetate (2 x 30 ml) and then the water removed in vacuo to give a pale yellowfoam. The foam was chromatographed on silica, eluting with ethyl acetate - ethanol - water (6:4:½). The fractions containing the title compound, Rf (ethyl acetate - ethanol - water; 5:4:½) = 0.46, were combined and the solvent removed under reduced pressure to give a pale yellow glass, which gave rise to a white solid when precipitated from acetone by the addition of ether (0.1 g; 42 %).

$\nu$ max (Nujol), 1790, 1690 (sh), 1620 $cm^{-1}$.

$\delta_H$ ($D_2O$) 5.66 (1H, d, J = 3Hz, H-$C_5$), 4.85 (1H, S, H-$C_3$) (2H, d, J = 7Hz, $H_2$-$C_9$), 3.51 (1H, dd, J = 3Hz and 17 Hz, αH-$C_6$), 2.99 (1H, d, J = 17Hz, βH-$C_6$), 2.95 (2H, d, J = 7 Hz, $CH_2CH(CH_3)_2$), 2.62 (3H, S, N-$CH_3$), 1.85 (1H, broad m, $CH(CH_3)_2$), 0.80 (6H, d, J = 7Hz, $CH(CH_3)_2$).

Example 8

Lithium 9-N-(isobutyl)-N-(benzoyl)aminodeoxyclavulanate

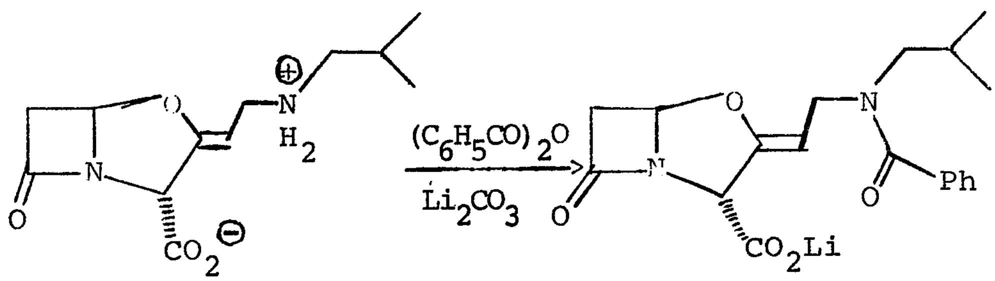


9-N-isobutylaminodeoxyclavulanic acid (0.5g, 1.9 mmol) was dissolved in dimethylformamide (25 ml) containing water (15 ml) and lithium carbonate (0.07g). After stirring at room temperature for 1/4 hour the reaction mixture was cooled to 0°C in an ice bath and a further portion of lithium carbonate (0.07g) added followed by benzoic anhydride (0.45g, 2 mmol). The reaction mixture was stirred at 0°C for a further 1½ hours, then the dimethylformamide was removed under reduced pressure to yield a yellow/brown solid. The solid was chromatographed on silica, eluting with ethyl acetate - ethanol - water (8:2:1). Fractions containing the required product, Rf ($SiO_2$/ethyl acetate : ethanol : water, 5:4:½) = 0.7, were combined and the solvent removed under reduced pressure. The rotational isomers of the title compound were obtained as a white solid by precipitation from acetone with ether (0.49g, 68%).

$\nu$max (KBr). 1785, 1690 (sh), 1600 $cm^{-1}$, (Nujol) 1785, 1790, 1610 $cm^{-1}$.

$\delta_H$ 35°C ($D_2O$); 7.34 (5H, m, NCOPh.), 5.68 and 5.44 (1H, 2 x d, J ; 3Hz, $H-C_5$), 4.90 and 4.72 (1H, 2 x s, $H-C_3$), 4.12 and 3.85 (2H, 2 x d, J ; 7Hz, $H_2-C_9$), 3.50 and 3.40 (1H, 2 x dd, J ; 3Hz and 17 Hz, $\alpha H-C_6$), 3.25 and 3.02 (2H, 2 x d, J ; 7Hz$CH_2(CH_3)_2$), 2.94 and 2.72 (1H, 2 x d, J ; 17Hz, $\beta H-C_6$), 1.95 (1H, broad m, $CH(CH_3)_2$), 0.9 and 0.6 (6H, 2 x d, J ; 7Hz, $CH(CH_3)_2$).

Example 9

Lithium 9-N-(isobutyl)-N-(acetyl)aminodeoxyclavulanate

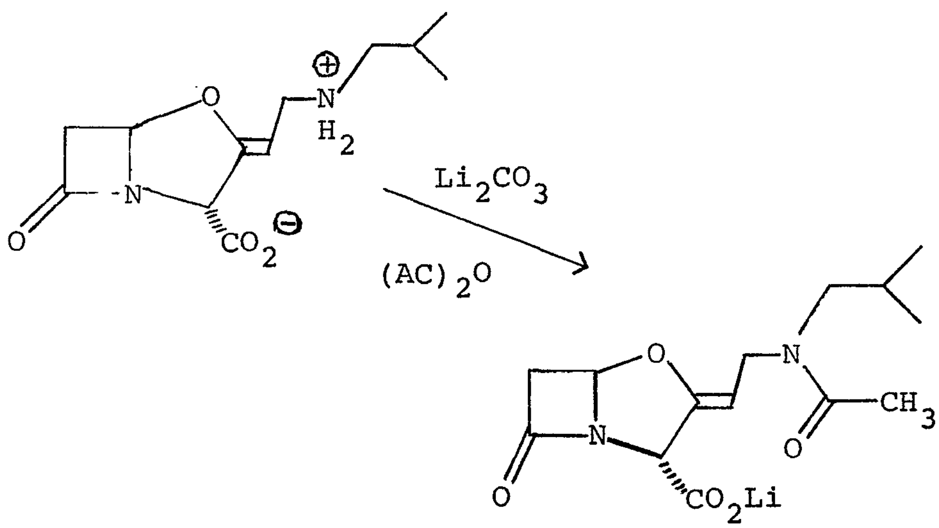


9-N-isobutylaminodeoxyclavulanic acid (0.34g; 1.3 mmol) was stirred at room temperature in dimethylformamide (10 ml) containing water (5 ml) and lithium carbonate (0.05g, 0.6 mmol). The mixture was stirred at room temperature for 1/4 hour, then filtered and evaporated in vacuo to yield lithium 9-N-isobutylaminodeoxyclavulanate as a white solid which was crystallised from acetone (0.29 g. 83%).

To a suspension of lithium 9-N-isobutylaminodeoxyclavulanate (0.29g; 1.1 mmol) in dry redistilled dimethylformamide (10 ml) was added acetic anhydride (0.12 ml; 1.2 mmol) and lithium carbonate (0.04g; 0.5 mmol). The reaction mixture was stirred at room temperature for ½ hour, then evaporated in vacuo to give a pale yellow solid. The solid was subjected to

silica gel column chromatography, eluting with ethyl acetate; ethanol : water : (6:4:½). The fractions containing the desired product, Rf (ethyl acetate : ethanol : water, 5:4:1) = 0.65 were combined and the solvent removed under reduced pressure to yield the title compound as a colourless glass obtained as a white solid when precipitated from acetone by the addition of ether (0.22g, 73%).

$\nu_{max}$ (Nujol) 1790, 1690 and 1610 $cm^{-1}$, ($CHCl_3$) 1790, 1690, 1610 $cm^{-1}$.

$\delta$ ($D_2O$) 0.80, 0.86 (3H, 2 x d, J 7Hz, $CH(C\underline{H}_3)_2$), 1.90 (1H, m, $C\underline{H}(CH_3)_2$), 2.06 and 2.06 (3H, 2 x s, $COC\underline{H}_3$) 3.02 (1H, d, J 17Hz, 6β-C$\underline{H}$, 3.10 3.15 (2H, 2 x d, J 7Hz, $C\underline{H}_2CH(CH_3)_2$), 3.55 (1H, dd, J 3 and 17 Hz, 6 α-C$\underline{H}$); 4.0, 4.05 (2H, m, 9-C$\underline{H}_2$), 4.87 (1H, m, 3-C$\underline{H}$), 5.70 (1H, m, 5-C$\underline{H}$).

Example 10

Lithium 9-N-(N-isobutyl-N'-methylthioureido) deoxyclavulanate

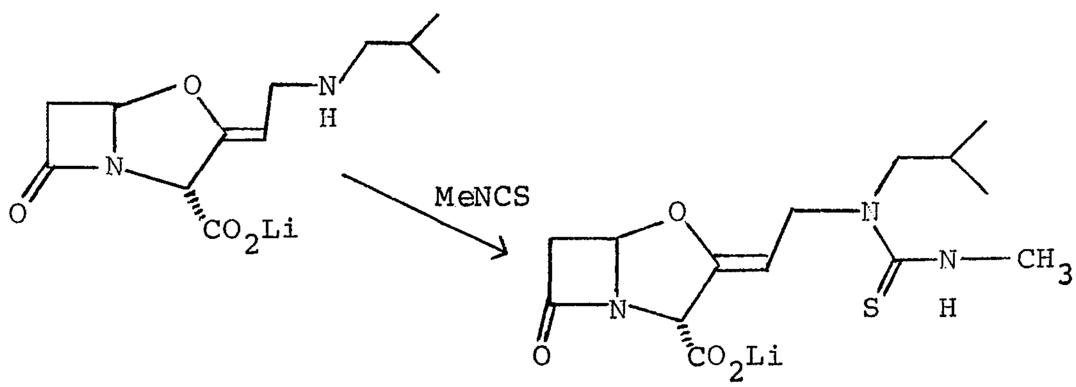


9-N-isobutylaminodeoxyclavulanic acid (0.51g; 2.0 mmol) was stirred at room temperature in dimethylformamide (10 ml) containing water (10 ml) and lithium carbonate (0.077g; 0.98 mmol) for 1/4 hour. The reaction mixture was then filtered and evaporated *in vacuo* to give lithium 9-N-isobutylaminodeoxyclavulanate, crystallised from acetone as colourless needles (0.49g, 95%).

To a stirred suspension of lithium 9-N-isobutylaminodeoxyclavulanate (0.24g, 0.92 mmol) in dry redistilled dimethylformamide (15 ml) at room temperature was added methylisothiocynate (0.07g, 0.95 mmol). The reaction mixture was stirred at room temperature for 24 hours then poured on to water (50 ml). The aqueous layer was washed with ethyl acetate (2 x 60 ml) then evaporated *in vacuo* to yield a yellow glass. This glass was subjected to silica gel column chromatography; eluting with ethyl acetate; ethanol: water (5:1:½), combining fraction Rf = 0.58 (ethyl acetate : ethanol :

water 2:1:½) and removal of the solvent gave a pale yellow glass which yielded the title compound as an off-white solid when precipitated from acetone by addition of ether (0.15 g, 53%).

$\nu_{max}$ ($CHCl_3$) 1790, 1660 and 1610 $cm^{-1}$.

$\delta$ ($D_2O$) 0.86 (6H, d, J 7Hz, $CH(C\underline{H}_3)_2$), 2.0 (1H, m, $C\underline{H}(CH_3)_2$), 2.49 (3H, s, $\underline{CH_3}N-\overset{\overset{S}{\|}}{C}$), 3.02 (1H, d, J 17Hz, 6β-C$\underline{H}$), 3.36 (2H, d, J 7Hz, $C\underline{H}_2CH(CH_3)_2$), 3.54 (1H, dd, J3 and 17 Hz, 6α-C$\underline{H}$) 4.27 (2H, m, 9-$CH_2$), 4.87 (1H, s, 3-C$\underline{H}$), 5.68 (1H, d, J 3 Hz, 5-C$\underline{H}$).

## BIOLOGICAL DATA

In a standard MIC synergy test, the following data were obtained for the compounds of this invention.

| Amoxycillin + the compound of Example at | μg/ml | MIC μg/ml amoxycillin Staph. aureus Russell | Kleb. aerogenes E70 | Escherichia coli JT39 |
|---|---|---|---|---|
| 4 | 5.0<br>1.0 | 0.3<br>0.6 | 50<br>100 | 62.5<br>500 |
| 2 | 5.0<br>1.0 | 0.3<br>0.6 | >100<br>>100 | 500<br>>500 |
| 3 | 5.0<br>1.0 | 0.08<br>0.6 | 3.1<br>12.5 | 2.0<br>16.0 |
| 1 | 5.0<br>1,I | 0.08<br>0.3 | 3.1<br>12.5 | 8.0<br>31.2 |
| 5 | 5.0<br>1.0 | 0.04<br>0.4 | >100<br>>100 | 250<br>250 |
| 8 | 5.0<br>1.0 | -<br>0.3 | >100<br>>100 | 125<br>>500 |
| 7 | 5.0<br>1.0 | 0.16<br>0.6 | 50<br>>100 | 31.2<br>500 |
| Amoxycillin alone | | 500 | 1000 | 2000 |

CLAIMS:

1. A compound of formula (I):

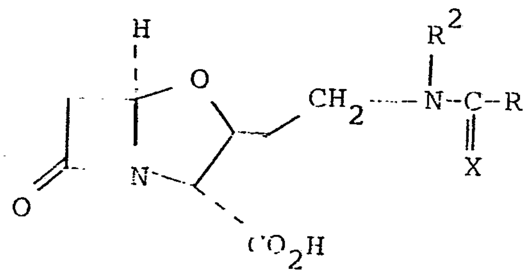

(I)

or a salt or ester thereof; wherein R represents a group $R^1$, $-OR^1$, $SR^1$ or $NR_AR_B$, where $R^1$, $R_A$ and $R_B$ independently represent hydrogen, a substituted or unsubstituted hydrocarbon radical, or $R_A$ and $R_B$ together form the residue of a heterocyclic ring, X represents oxygen or sulphur and $R^2$ is an optionally substituted hydrocarbon or heterocyclic group.

2. A compound as claimed in claim 1 in the form of the free acid, pharmaceutically acceptable salt or in vivo hydrolysable ester thereof.

3. A compound as claimed in claim 1 or 2 wherein X represents oxygen.

4. A compound as claimed in any one of claims 1 to 3 wherein $R^2$ represents a $C_{1-6}$ group optionally substituted by hydroxy, carboxy, cyano or a carboxylic acid, salt or ester.

5. A compound as claimed in any one of claims 1 to 4 wherein R represents hydrogen or an optionally substituted hydrocarbon radical of up to 20 carbon atoms.

6. A compound as claimed in any one of claims 1 to 5 wherein R represents hydrogen, $C_{1-6}$ alkyl, $C_{1-6}$ alkoxy, $C_{1-6}$ alkylamino or phenyl optionally substituted with from 1 to 3 substituents selected from $C_{1-6}$ alkyl, nitro halogen or hydroxy.

7. A compound as claimed in claim 6 wherein R represents hydrogen.

8. A process for the preparation of a compound as claimed in claim 1, which process comprises reacting a compound of formula (IV):

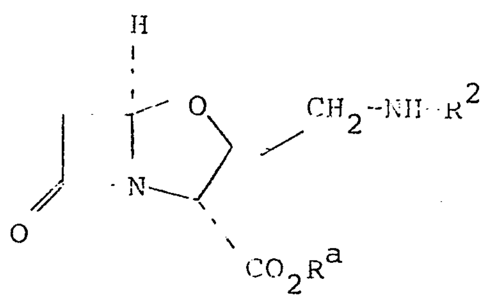

(IV)

wherein $R^2$ is as defined in claim 1 and $R^a$ is a hydrogen atom or a carboxy-blocking group; with an N-acylating derivative of an acid of formula:

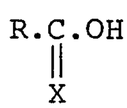

wherein R and X are as defined in claim 1;

and optionally thereafter:

i) removing any carboxy-blocking group $R^a$;

ii) converting the product into a salt or ester.

9. A pharmaceutical composition which comprises a pharmaceutically acceptable carrier and a compound as claimed in claim 2.

10. A composition as claimed in claim 9 which further comprises an antibacterial agent.

European Patent Office

# EUROPEAN SEARCH REPORT

0068617

Application number

EP 82 30 2483

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | DE-A-2 747 599 (GLAXO)<br>*Claims 1-9,24-27*<br>--- | 1,8-10 | C 07 D 498/04<br>A 61 K 31/42 //<br>(C 07 D 498/04<br>C 07 D 263/00<br>C 07 D 205/00 )<br>(A 61 K 31/42<br>A 61 K 31/43 )<br>(A 61 K 31/42<br>A 61 K 31/545 ) |
| Y | US-A-4 258 050 (J.B.HARBRIDGE)<br>*Claims 1,2,59,60*<br>--- | 1,9 | |
| P,Y | GB-A-1 604 823 (BEECHAM)<br>*Page 1, lines 16-18; claims*<br>----- | 1,8-10 | |
| | | | TECHNICAL FIELDS SEARCHED (Int. Cl. 3) |
| | | | C 07 D 498/00<br>A 61 K 31/00 |

The present search report has been drawn up for all claims

| Place of search | Date of completion of the search | Examiner |
|---|---|---|
| THE HAGUE | 14-09-1982 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82